# EUROPÄISCHE PATENTSCHRIFT

(11) **EP 0 243 298 B1**
(45) Veröffentlichungstag und Bekanntmachung des Hinweises auf die Patenterteilung: **31.03.1993**
(21) Anmeldenummer: 87730042.6
(22) Anmeldetag: 21.04.1987
(51) Int. Cl.: A61F 2/36, A61F 2/30, A61F 2/28

(54) **Bausatz für eine Schaftprothese**
Construction kit for a stem prosthesis
Eléments de construction d'une prothèse fémorale avec tige

(30) Priorität: 25.04.1986 DE 8611697 U
(43) Veröffentlichungstag der Anmeldung: 28.10.1987
(73) Patentinhaber: Johnson & Johnson Professional Products GmbH, 22848 Norderstedt (DE)
(72) Erfinder: Kranz, Curt, D-1000 Berlin 62 (DE); Anapliotis, Emmanuel, D-1000 Berlin 33 (DE)
(74) Vertreter: UEXKÜLL & STOLBERG

(56) Entgegenhaltungen:
- DE-A- 3 138 848

## Beschreibung

Die Erfindung betrifft einen Bausatz der im Oberbegriff des Anspruchs 1 angegebenen Art.

Ein solcher Bausatz ist bekannt aus der DE-A-32 05 577. Die dort beschriebenen Teilstücke, die zu einer Femurprothese gewünschter Länge zusammensteckbar sind, ergeben zusammengesetzt einen relativ starren, gerade verlaufenden Femurersatz.

Die bekannte Schaftprothese hat den Nachteil, daß beim Implantieren der zusammengesetzten Prothese eine Lockerung der Verbindungselemente (Außen- und Innenkonus) auftritt, welche dazu führt, daß möglicherweise die Gefahr einer Relativdrehung der Einzelteile um die Längsachse des Schaftes möglich ist.

Der im Anspruch 1 angegebenen Erfindung liegt die Aufgabe zugrunde, eine Möglichkeit zu schaffen, nachträglich die Einzelteile der Prothese durch Kompression sicher miteinander zu verbinden, so daß sie auch bei während der Benutzung auftretenden Stößen etc. sicher miteinander verbunden bleiben.

Insbesondere ist aber günstig, daß der zum Setzen der Verbindungskonen herangezogene Zuganker nach dem Erzeugen der festen Verbindung vollständig aus dem Prothesenschaft entfernt werden kann, so daß dort keinerlei Teile verbleiben, welche sich lösen oder mit der Körperflüssigkeit unerwünschte Reaktionen zeigen können. Mit dem Zuganker gemäß der Erfindung können beliebige Anzahlen von aufeinanderfolgenden Elementen miteinander verbunden werden, so daß auch bei Reoperationen ohne Entfernung des Endes ein einzufügendes Zwischenteil nach dem gleichen Prinzip befestigt werden kann.

Bei einer günstigen Ausführung der Erfindung ist der Zuganker so lang gewählt, daß er auch bei der maximalen Anzahl von zusammenzufügenden Elementen aus dem Prothesenoberteil, d.h. dem Kopfteil, soweit herausragt, daß nach Abriß seines Endes das verbleibende Schaftende erfaßt, herausgedreht und aus der Prothese entfernt werden kann. Zum Verbinden des Zugankers mit dem letzten (am tiefsten gelegenen) Prothesenelement, d.h. mit dem Endteil, sind solche Verbindungen geeignet, die die Übertragung von Zugkräften ermöglichen. Dazu gehören insbesondere Schraubgewinde, Bajonettkupplungen oder aufspreizbare Enden des Zugankers, welche nach Art von Widerhaken quer zur Zugrichtung stehende Flächenbereiche hintergreifen.

Eine Hülse unterschiedlicher Länge ermöglicht die Übertragung von Druckkräften in das Prothesenoberteil (oder das oberste zu verbindende Prothesenelement), wobei das Ende der Hülse als widerlager für auf den Zuganker auszuübende Kräfte gilt. Zum Ausüben von Zugkräften ist eine Mutter oder eine entsprechend ausgebildete Zange geeignet. Das nach Abreißen des Endes des Zugankers oberhalb der Sollbruchstelle zunächst im Prothesenschaft verbleibende Ende des Zugankers weist ein Gewinde auf, dessen Gangrichtung entgegengesetzt zur Löserichtung des im untersten zu verbindenden Prothesenelement vorgesehenen Gewindes oder anderer Verbindungen gerichtet ist, so daß beim Aufdrehen eines Lösewerkzeugs mit Innengewinde durch Aufdrehen sich das Ende des Zugankers im untersten Prothesenteil löst.

Eine vorteilhafte Weiterbildung der Erfindung besteht darin, daß das Endteil eine Verstelleinrichtung aufweist, mit der bewegliche Elemente des Endteils nach dem Einsetzen der Bausatz-Schaftprothese auseinander drückbar und mit der Markhöhlenwandung des schaftaufnehmenden Knochens verklemmbar sind.

Damit ist der Einsatz einer Bausatzprothese auch für sehr lange Schaftprothesen möglich, wobei die Bausatzprothese genau und verdrehungssicher implantierbar ist und im implantierten Zustand an unterschiedliche Markhöhlenquerschnitte des schaftaufnehmenden Knochens anpaßbar ist, so daß sie fest im Markkanal verankert werden kann, daß aber gleichzeitig die Möglichkeit offen bleibt, im Falle einer notwendigen Reimplantation die Bausatzprothese leicht und ohne wesentliche Beschädigung des Knochens aus dem Markkanal herauszulösen.

In einer weiteren Ausgestaltung der erfindungsgemäßen Lösung bestehen die beweglichen Elemente des Innenteiles, d.h. des Endteils, aus auseinander spreizbaren Elementen mit in axialer Richtung veränderlichem Querschnitt, zwischen denen ein Bolzen oder Keil angeordnet ist, der über ein in der durchgehenden Bohrung angeordnetes Spannelement in axialer Richtung beweglich ist, so daß bei Ausüben einer axialen Kraft auf den Bolzen oder Keil die Elemente auseinander gespreizt werden. Die Verbindung des Spannelementes mit dem Bolzen oder Keil kann dauerhaft oder als Schraubgewindeverbindung, Bajonettkupplung oder dergleichen ausgestaltet sein. Die Spreizrichtung der auseinander spreizbaren Elemente kann je nach Art und Länge der Schaftprothese sowie Ausbildung des Markkanals in oder entgegengesetzt zur Einschubrichtung der Schaftprothese angeordnet werden, so daß sich die auseinander spreizbaren Elemente entweder der sich erweiternden Markhöhlenwandung anlegen oder nach Art von Widerhaken sich im Markkanal verankern.

Eine weitere vorteilhafte Ausgestaltung der erfindungsgemäßen Lösung besteht darin, daß die beweglichen Elemente des Endteils aus federnden Elementen bestehen, die am oberen und unteren Ende des Endteils miteinander verbunden sind und daß das Spannelement am unteren Ende der federnden Elemente befestigbar ist, derart, daß bei einer Bewegung des Spannelementes in axialer Richtung die federnden Elemente auseinander biegbar sind.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der in der Zeichnung dargestellten Figuren näher erläutert.

Es zeigen:
Figur 1 ein Ausführungsbeispiel eines ersten Bausatzes einschließlich eines die Gelenkkugel tragenden Kopfteils,
Figur 1a bis 1b Zubehörteile des Bausatzes gemäß Figur 1,
Figur 2 eine teilweise im Längsschnitt dargestellte Bausatzprothese mit einem bewegliche Elemente aufweisenden Endteil;
Figur 3 ein im Längsschnitt dargestelltes vergrößertes Endteil der Bausatzprothese gemäß Figur 2 mit auseinander spreizbaren beweglichen Elementen;
Figur 4 ein im Längsschnitt dargestelltes vergrößertes Endteil der Bausatzprothese gemäß Figur 2 mit federnden Elementen;
Figur 5 das in Figur 4 dargestellte Endteil mit federnden Elementen im gespannten Zustand;
Figur 6 einen Längsschnitt durch ein Endteil einer Bausatzprothese mit entgegen der Einschubrichtung auseinander spreizbaren beweglichen Elementen; im ungespreizten Zustand und
Figur 7 das in Figur 6 dargestellte Endteil mit gespreizten beweglichen Elementen.

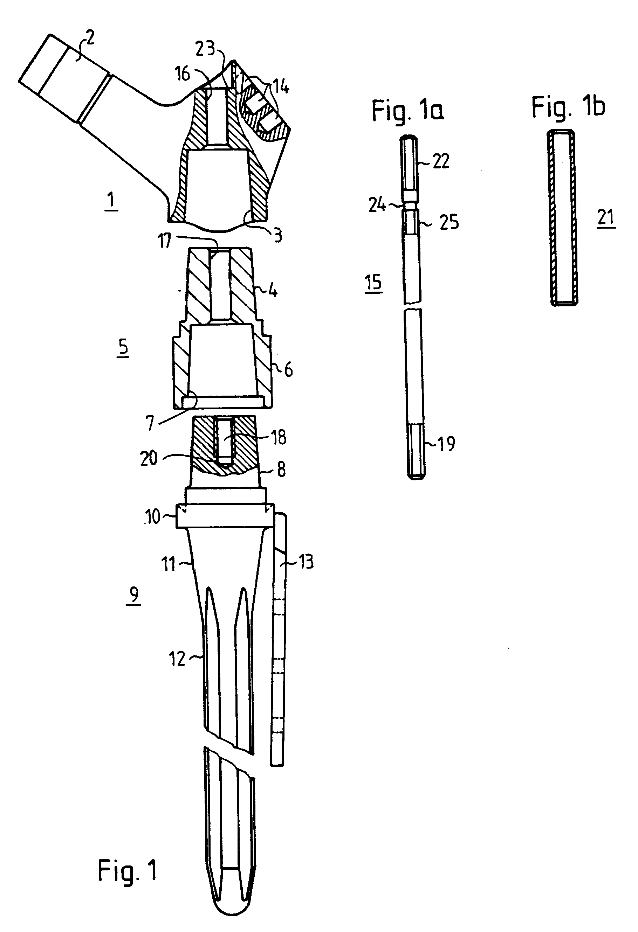

In Figur 1 ist für ein Kopfteil 1 einer Femurprothese ein Konus 2 für eine aufsteckbare Gelenkkugel vorgesehen. Am unteren Ende weist das Kopfteil 1 eine konische Bohrung 3 auf, in welche ein Zapfen 4 eines Zwischenteiles 5 mit kreiszylindrischem Rumpf 6 und einer konischen Bohrung 7 einsetzbar ist.

In die konische Bohrung 7 wiederum paßt ein Zapfen eines - nicht dargestellten - weiteren Zwischenteiles, das im wesentlichen wie das Zwischenteil 5 aufgebaut ist, jedoch auch abweichende Längen aufweisen kann. Mehrere solcher Zwischenteile 5 können zu einem Schaft entsprechend der in Figur 1 gezeigten Prothese zusammengefügt werden.

In das untere Ende eines Zwischenteils - nämlich in eine entsprechende konische Bohrung 7 des letzten Zwischenteiles 5 - ist ein konischer Zapfen 8 eines Endteils 9 steckbar. Das Endteil 9 weist zunächst einen Bereich 10 mit zylindrischem Querschnitt auf, der an den Außenquerschnitt der Zwischenteile 5 bzw. des Kopfteils 1 angepaßt ist. Ein konischer Ansatz 11 mit einem im wesentlichen zylindrisch ausgebildeten Schaftende 12 mit gegenüber dem Zwischenteil verringertem Durchmesser ist dazu bestimmt, in den Markraum einzementiert oder verankert zu werden. Das Unterteil 9, d.h. das Endteil, der Prothese weist weiterhin eine senkrecht zum Schaftende hin gerichtete Zunge 13 auf, welche Bohrungen zum zusätzlichen Befestigen des Schaftes aufweist. Das Oberteil 1 ist mit Bohrungen 14 versehen, welche zur Befestigung der Bänder dienen.

Die Elemente 1, 5 und 9 werden in konventioneller Weise zusammengesetzt und die zusammengesetzte Prothese implantiert, wobei gegebenenfalls der Zuganker 15 bereits eingefügt ist. Im Bedarfsfall kann aber auch das das Schaftende 12 aufweisende Unter teil 9 allein oder mit einem oder mehreren Zwischenstücken 5 befestigt und schließlich das Oberteil 1 aufgesetzt werden, wobei der Zuganker 15 ebenfalls nachträglich eingesetzt werden kann.

Im Durchlaß des Zugankers 15 weisen die Prothesenteile 1, 5 und 9 jeweils durchgehende Bohrungen 16 und 17 bzw. eine Sackbohrung 18 im Unterteil 9 auf, welche bei zusammengesetzter Prothese axial zueinander ausgerichtet sind. Der (in Figur 1a separat dargestellte) Zuganker 15 wird mit seinem ein Außengewinde 19 aufweisenden Ende durch die Bohrungen 16 und 17 des Ober- bzw. des Zwischenteils der Prothese hindurch in das Innengewinde 20 der Sackbohrung 18 eingedreht, so daß er Zugkräfte auf das Untereil 9 der Schaftprothese ausgeben kann. Das freie Ende des Zugankers 15 überragt im eingesetzten Zustand das Oberteil der Prothese so weit, daß - auch wenn die in Figur 1b dargestellte Hülse 21 auf das aus dem Prothesenoberteil herausragende Ende gesetzt ist - das obere Ende des Zugankers 15, welches ebenfalls mit einem Außengewinde 22 versehen ist, aus der Hülse 21 herausragt.

Das untere Ende der Hülse 21 stützt sich an einer die Bohrung 16 im Oberteil 1 der Prothese umgebenden Auflagekante 23 ab, welche auf den Querschnitt der Hülse 21 abgestimmt ist. Der Zuganker 15 weist in der Nähe des aus dem Oberteil 1 der Prothese herausragenden Endes eine Einschnürung 24 auf, die als Sollbruchstelle dient.

Die Zugkräfte werden auf den Zuganker 15 durch ein Element aufgebracht, welches sich auf dem oberen Ende der Hülse 21 abstützt. Geeignet ist dazu eine Mutter, welche auf das Außengewinde 22 aufgedreht wird, ober eine Zange, die in ihrer Art für Blindniete bekannt ist. Um einheitliche Zuganker verwenden zu können, ist es vorteilhaft, wenn Hülsen 21 mit unterschiedlicher Länge vorrätig gehalten werden, welche je nach der herzustellenden Prothesenlänge ausgewählt werden und den Überstand des Zugankers 15 über das Oberteil 1 ausgleichen. Der Satz der Hülsen 21 mit unterschiedlicher Länge braucht dabei nur einmal angeschafft zu werden und es sind auch nur einheitliche Längen der Zuganker 15 als Verschleißteile notwendig. Nach dem Abreißen des herausstehenden Endes des Zugankers 15 sind die konischen Zapfen bzw. die Bohrungen der Prothesenelemente hinreichend fest miteinander verbunden, so daß eine Lockerung bei Benutzung nicht zu befürchten ist. Nach Entfernen der Hülse 21 kann das verbleibende obere Ende des Zugankers 15 ergriffen werden und von der Prothese gelöst werden. Dabei weist der Bereich unterhalb der Einschnürung 24 bevorzugt ein Gewinde 25 auf, dessen Gangrichtung entgegengesetzt zum Gewinde 19 ist, so daß ein auf das Gewinde 25 aufgebrachtes Schraubwerkzeug mit Innengewinde das verbleibende Ende des Zugankers 15 durch Schrauben aus dem Innengewinde 20 des Unterteils herauslöst und zusammen mit dem Zuganker entfernt werden kann.

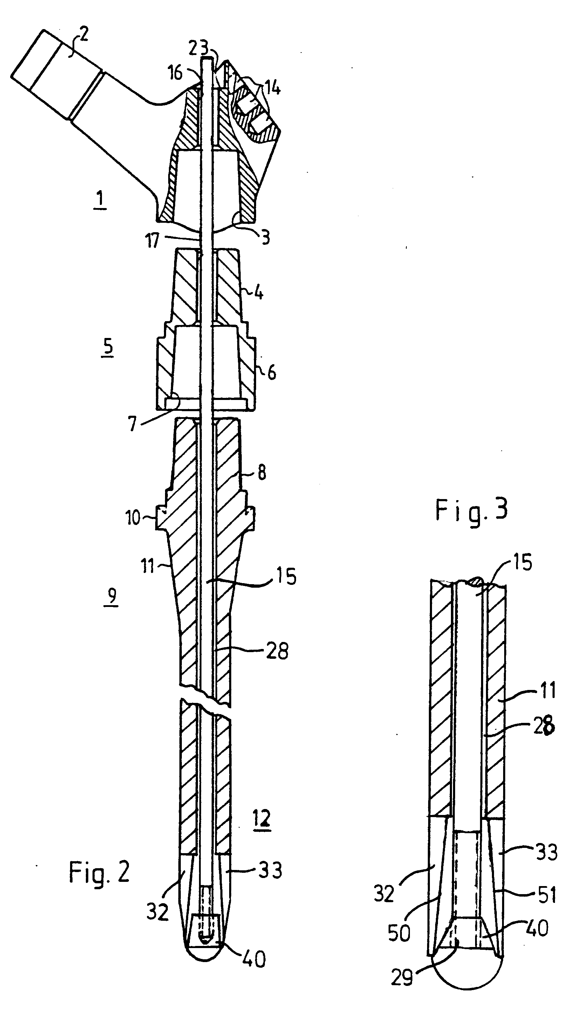

Die in Figur 2 dargestellte Bausatzprothese entspricht weitgehend der Bausatzprothese gemäß Figur 1, wobei gleiche Bezugsziffern gleiche Teile wie in Figur 1 bezeichnen.

Das im wesentlichen zylindrisch ausgebildete Schaftende 12 der Bausatzprothese gemäß Figur 2 weist am unteren Ende bewegliche Schaftteile 32, 33 auf, die mittels einer radial auf sie wirkenden Kraft auseinander gedrückt werden können. Diese radiale Kraft wird mittels einer Verstelleinrichtung bewirkt, die im vorliegenden Ausführungsbeispiel aus einem Keil 40 in Verbindung mit einem Zuganker 15 als Spannelement aufgebracht wird.

Der Zuganker 15 wird nach dem Zusammensetzen der einzelnen Elemente 1, 5 und 9 der Bausatzprothese und wahlweise vor oder nach der Implantation der zusammengesetzten Prothese in durchgehende Bohrungen 16, 17, 28 im Kopfteil 1, Zwischenteil 5 und Endteil 9 eingefügt, wobei die durchgehenden Bohrungen 16, 17, 28 axial zueinander ausgerichtet sind.

Zu diesem Zweck wird der Zuganker 15 mit seinem ein Außengewinde 19 aufweisenden Ende durch die Bohrungen 16, 17, 28 des Ober-, Zwischen- und Endteils der Bausatzprothese hindurch in das Innengewinde 29 des Keils 40 eingedreht.

Das freie Ende des Zugankers 15 überragt im eingesetzten Zustand das Kopfteil der Bausatzprothese so weit, daß auch bei auf das Kopfteil 1 der Bausatzprothese aufgesetzter Hülse 21 das obere Ende des Zugankers 15, das ebenfalls mit einem Außengewinde 22 versehen ist, aus der Hülse 21 herausragt.

Das untere Ende der Hülse 21 stützt sich an der die Bohrung 16 im Kopfteil 1 der Bausatzprothese umgebenden Auflagekante 23 ab, welche auf den Querschnitt der Hülse 21 abgestimmt ist. Der Zuganker 15 weist in der Nähe des aus dem Kopfteil 1 der Prothese herausragenden Ende eine Einschneidung 24 auf, die als Sollbruchstelle dient.

Die Zugkräfte werden auf den Zuganker 15 analog zur Beschreibung der Figur 1 durch ein Element aufgebracht, welches sich auf dem oberen Ende der Hülse 21 abstützt.

Mit dem Aufbringen einer Zugkraft auf den Zuganker 15 legt sich der Keil 40 - wie der vergrößerten Querschnittsdarstellung gemäß Figur 3 zu entnehmen ist - an die Innenseiten 50, 51 der beweglichen Schaftteile 32, 33 an, so daß zunächst das Kopfteil 1, oder die Zwischenteile 5 und das Unterteil 9 fest zusammengezogen werden, so daß eine Lockerung der einzelnen Prothesenteile bei Benutzung nicht zu befürchten ist. Wird die Zugkraft erhöht, indem beispielsweise die auf das Außengewinde 22 des Zugankers 15 aufgedrehte Mutter weitergedreht wird, so bewirkt die durch den Keil 40 auf die Innenflächen 50, 51 der beweglichen Schaftteile 32, 33 ausgeübte Kraft, daß die beweglichen Schaftteile 32, 33 radial auseinander gedrückt werden. Dabei werden die beweglichen Schaftteile 32, 33 so weit radial auseinander gedrückt, daß sie fest an der Markhöhlenwandung des schaftaufnehmenden Knochens anliegen.

Nach dem Abreißen des herausstehenden Endes des Zugankers 15 sind die konischen Zapfen bzw. Bohrungen 3, 4, 7, 8 der Prothesenelemente 1, 5, 9 fest miteinander verbunden und die beweglichen Schaftteile 32, 33 liegen fest an der Markhöhlenwandung an. Nach Entfernen der Hülse 21 kann analog zur Beschreibung der Figur 1 das verbleibende obere Ende des Zugankers 15 ergriffen und von der Prothese gelöst werden. Da der Keil 40 fest an den Innenseiten 50, 51 der beweglichen Schaftteile 32, 33 anliegt, ist ein Lösen und damit Nachgeben der beweglichen Schaftteile 32, 33 nicht zu befürchten, so daß ein fester Sitz gewährleistet ist. Alternativ hierzu kann selbstverständlich auch der Zuganker 15 in der Bausatzprothese verbleiben und mittels einer auch auf das Gewinde 25 aufgeschraubten Mutter arretiert werden.

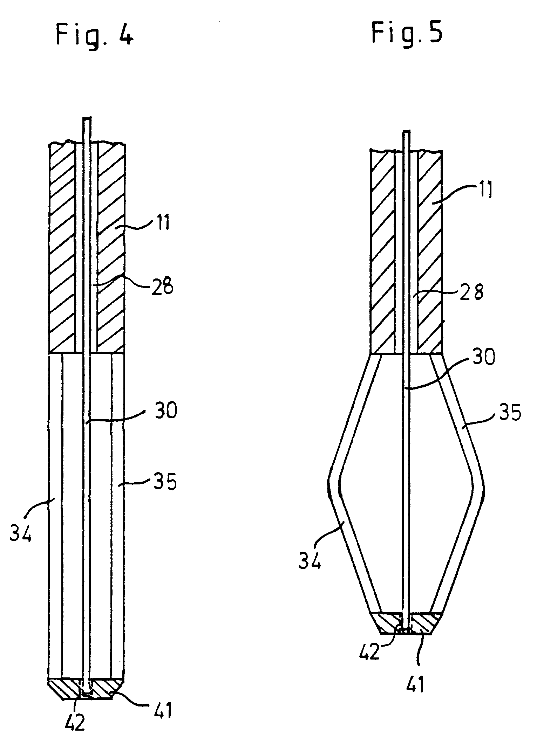

Das in den Figuren 4 und 5 dargestellte Schaftende 12 weist federnde Elemente 34, 35 auf, die sich bei Ausüben einer Zugkraft auf die unteren Enden der federnden Elemente 34, 35 auseinander drücken lassen. Die unteren Enden der federnden Elemente 34, 35 sind mittels eines Verbindungselementes 41 starr miteinander verbunden, wobei das Verbindungselement 41 beispielsweise Schlitze einer Bajonettkupplung enthält, in die die am unteren Ende des Spannelementes 30 vorgesehenen Bajonettstifte einsetzbar sind. Durch Ziehen an dem Spannelement 30, das als dünne Stange ausgebildet sein kann, werden die federnden Elemente 34, 35 entsprechend der in Figur 5 dargestellten Form gespannt und drücken im gespannten Zustand gegen die Markhöhlenwandung.

Bei der in Figur 4 und 5 dargestellten Ausführungsform des Schaftendes mit beweglichen Schaftteilen 34, 35 kann das Spannelement 30 beispielsweise in einer Bohrung des Zugankers 15 gemäß den Figuren 2 und 3 geführt werden, wobei das untere Außengewinde 19 des Zugankers 15 in ein im oberen Konus 8 des Endteils 9 vorgesehenes Innengewinde eingreift, so daß das Zusammendrücken des Kopfteils 1, des bzw. der Zwischenteile 5 und des Endteils 9 über den Zuganker 15 erfolgt, während hiervon getrennt das Auseinanderdrücken der beweglichen Schaftteile 34, 35 mittels des Spannelements 30 durchgeführt wird, das in das Innengewinde 42 des Verbindungselements 41 mit einem Außengewinde eingreift.

Anstelle einer dünnen Stange als Spannelement 30 ist auch die Verwendung eines Zugfadens möglich, der fest mit dem Verbindungselement 41 verbunden und in dem gespannten Zustand gemäß Figur 5 im Bereich des Kopfteils 1 befestigbar ist.

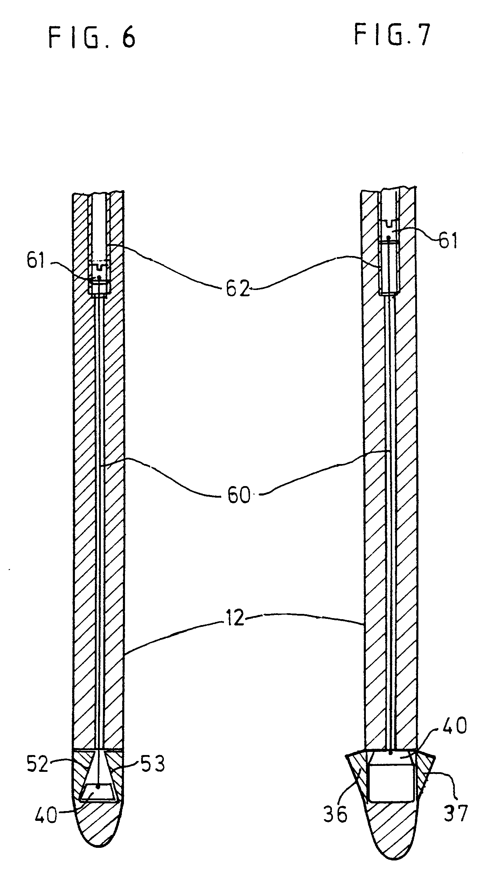

In den Figuren 6 und 7 ist ein weiteres Ausführungsbeispiel der vorliegenden Erfindung dargestellt, wobei Figur 6 das Schaftende dieses Ausführungsbeispieles im Längsschnitt mit eingeklappten beweglichen Schaftteilen und die Figur 7 das Schaftende ebenfalls im Längsschnitt mit ausgeklappten beweglichen Schaftteilen zeigt.

Die Innenflächen 52, 53 der beweglichen Schaftteile 36, 37 weisen einen sich vom unteren zum oberen Ende des Unterteils bzw. Schaftendes 12 verringernden Abstand auf, so daß bei der Bewegung eines Keils 40 vom unteren Ende in Richtung auf das obere Ende die beweglichen Schaftteile 36, 37 auseinander gedrückt werden. Da die beweglichen Schaftteile im Bereich des unteren Endes des Schaftendes 12 angelenkt sind, ergibt sich die in Figur 8 dargestellte Querschnittsform des Schaftendes im gespannten Zustand der beweglichen Schaftteile 36, 37. Die hierfür erforderliche Spannkraft kann beispielsweise mittels eines Zugfadens 60 aufgebracht werden, indem am oberen Ende eine in einem Gewinde 62 verstellbare Schraube 61 mit dem Zugfaden 60 verbunden ist und bei Herausdrehen der Schraube 61 aus dem Gewinde 62 der Keil 40 am unteren Ende in Richtung auf das obere Ende des Schaftendes 12 gezogen wird.

Das Herausdrehen der Schraube 61 kann mittels eines gesonderten Werkzeugs nach dem Verspannen der einzelnen Elemente 1, 5, 9 der Bausatzprothese und Herausnehmen des Zugankers 15 oder in der Weise erfolgen, daß der Zuganker 15 in ein im oberen Konus des Endteils 9 vorgesehenes durchgehendes Gewinde 18 eingreift, die Zugspannung auf den Zuganker 15 zum Zusammenfügen der Elemente 1, 5, 9 der Bausatzprothese aufgebracht und anschließend der Zuganker 15 weitergedreht wird, bis ein Eingriff mit der Schraube 61 beispielsweise über einen Außen- und Innensechskant hergestellt ist. Durch Zurückdrehen des Zugankers 15 wird die Schraube 61 mitgedreht und bewirkt über den Spanndraht 60 und den Keil 40 das Auseinanderdrücken der beweglichen Elemente 36, 37 und damit ein festes Verankern des Schaftendes der Bausatzprothese im Markkanal des schaftaufnehmenden Knochens.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Bausatz für eine Schaftprothese mit einem mit einer Gelenkkugel versehbaren Kopfteil (1) und einem im Knochenmerkraum verankerbaren Endteil (9), von denen das eine Teil einen konischen Zapfen (8) und das andere Teil eine konische Bohrung (3) aufweist und zwischen diesen beiden Teilen mindestens ein an Zapfen (8) und/oder Bohrung (3) angepaßtes Zwischenteil (5) vorgesehen ist,
**dadurch gekennzeichnet,**
daß das Kopfteil (1) und das Zwischenteil (5) eine durchgehende Bohrung (16; 17) und das Endteil (9) eine Bohrung (18, 28; 17) mit Mitteln (20; 29; 42; 62) zum lösbaren Eingriff zwecks Übertragung einer Kraft in axialer Richtung aufweisen, wobei die Bohrungen (16; 17; 18, 28) bei zusammengefügten Teilen (1; 5; 9) axial zueinander in Richtung des Schaftes ausgerichtet sind, und
daß ein Zuganker (15) mit einem Eingriffselement zum lösbaren Eingriff in die Mittel (20; 29; 42; 62) vorgesehen ist und der Zuganker (15) bei zusammengefügten Prothesenteilen das Kopfteil (1) überragt.

2. Bausatzprothese nach Anspruch 1, **dadurch gekennzeichnet,** daß die Mittel zum lösbaren Eingriff und das entsprechende Eingriffselement des Zugankers (15) ein Innengewinde (20; 29; 42; 62) bzw. ein Außengewinde (19), die beiden Teile einer Bajonettkupplung oder mindestens ein aus dem Zuganker (15) ausspreizbares Element mit einer entsprechenden Gegenfläche bilden.

3. Bausatzprothese nach einem der vorangehenden Ansprüche, **dadurch gekennzeichnet,** daß das Kopfteil (1) und/oder mindestens ein Zwischenteil (5) an der einem Konus (3 bzw. 7) oder einer Bohrung (16 bzw. 17) gegenüberliegenden Seite eine Anlagefläche (23) aufweist, die die Öffnung der Bohrung (16 bzw. 17) umgibt.

4. Bausatzprothese nach Anspruch 3, **dadurch gekennzeichnet,** daß eine auf den Zuganker (15) aufschiebbare Hülse (21) vorgesehen ist, deren Querschnitt an die Anlagefläche (23) angepaßt ist.

5. Bausatzprothese nach einem der vorangehenden Ansprüche, **dadurch gekennzeichnet,** daß der Zuganker (15) an seinem dem Eingriffselement gegenüberliegenden Ende ein Außengewinde (22) aufweist.

6. Bausatzprothese nach einem der vorangehenden Ansprüche, **dadurch gekennzeichnet,** daß der Zuganker (15) einen Bereich (24) mit verringertem Querschnitt aufweist, wobei die Zugfestigkeit dieses Bereichs einer Kraft entspricht, die ausreichend ist, um die Verbindungskonen der Kopf-, End- und Zwischenteile (1, 5 und 9) derart zu setzen, daß sie gegen Lockerung im implantierten Zustand gesichert sind.

7. Bausatzprothese nach Anspruch 6, **dadurch gekennzeichnet,** daß nahe dem Bereich mit verringertem Querschnitt am längeren Ende des Zugankers (15) ein Außengewinde (25) vorgesehen ist.

8. Bausatzprothese nach Anspruch 7, **dadurch gekennzeichnet,** daß das als Außengewinde (19) am Zuganker (15) ausgebildete Eingriffselement eine entgegengesetzte Gangrichtung zum Außengewinde (25) aufweist.

9. Bausatzprothese nach einem der vorstehenden Ansprüche **dadurch gekennzeichnet,** daß das Endteil (9) eine Verstelleinrichtung (40; 30, 41; 40, 60, 61) aufweist, mit der bewegliche Elemente (32, 33; 34, 35; 36, 37) des Endteils (9) nach dem Einsetzen der Schaftprothese auseinander drückbar und mit der Markhöhlenwandung des schaftaufnehmenden Knochens verklemmbar sind.

10. Bausatzprothese nach Anspruch 9, **dadurch gekennzeichnet,** daß die Verstelleinrichtung ein in der durchgehenden Bohrung (16, 17, 28) angeordnetes Spannelement (30) zur Übertragung einer Kraft in axialer Richtung aufweist.

11. Bausatzprothese nach Anspruch 9 oder 10, **dadurch gekennzeichnet,** daß die beweglichen Elemente des Endteils (9) aus auseinander spreizbaren Schaftteilen (32, 33; 36, 37) mit in axialer Richtung veränderlichem Querschnitt bestehen, und daß das Spannelement (30) mit einem zwischen den auseinander spreizbaren Schaftteilen (32, 33; 36, 37) angeordneten, in axialer Richtung beweglichen Bolzen oder Keil (40) verbunden ist.

12. Bausatzprothese nach Anspruch 11, **dadurch gekennzeichnet,** daß die auseinander spreizbaren Schaftteile (32, 33) am freien Schaftende (12) auseinander spreizbar sind.

13. Bausatzprothese nach Anspruch 11, **dadurch gekennzeichnet,** daß die auseinander spreizbaren Schaftteile (36, 37) entgegen der Einführungsrichtung des Schaftendes (12) auseinander spreizbar sind.

14. Bausatzprothese nach Anspruch 9 oder 10, **dadurch gekennzeichnet,** daß die beweglichen Elemente des Endteils (9) aus federnden Elementen (34, 35) bestehen, die am oberen und unteren Ende des Endteils (9) miteinander verbunden sind und daß das Spannelement (30) am unteren Ende der federnden Elemente (34, 35) befestigbar ist, derart, daß bei einer Bewegung des Spannelementes (30) in axialer Richtung die federnden Elemente (34, 35) auseinander biegbar sind.

15. Bausatzprothese nach einem der vorstehenden Ansprüche 9 bis 14 **dadurch gekennzeichnet,** daß das Spannelement (30) aus einem durch eine Bohrung im Zuganker (15) geführten Zugfaden oder -draht besteht, der im Bereich des Kopfteils (1) der Schaftprothese arretierbar ist.

16. Bausatzprothese nach einem der vorstehenden Ansprüche 9 bis 14, **dadurch gekennzeichnet,** daß das Spannelement (30) aus einer Zug- oder Druckstange besteht, die mit einem im Bolzen oder Keil (40) vorgesehenen Mittel zum lösbaren Eingriff (29) verbindbar ist.

17. Bausatzprothese nach Anspruch 14, **dadurch gekennzeichnet,** daß das Spannelement (30) aus einer Zugstange besteht, die mit einem am unteren Ende der federnden Elemente (34, 35) befestigbaren Mittel zum lösbaren Eingriff (42) verbindbar ist.

18. Bausatzprothese nach einem der vorstehenden Ansprüche **dadurch gekennzeichnet,** daß das Spannelement (30) der Zuganker (15) ist.

## Claims

1. Kit for a shaft prosthesis, having a top part (1), which can be provided with a joint ball, and an end part (9) which can be anchored in the marrow space, the one part having a conical pin (8) and the other part having a conical bore (3), and at least one intermediate part (5) which is adapted to the pin (8) and/or the bore (3) being provided between said top and end parts, characterised in that the top part (1) and the intermediate part (5) have a through-bore (16; 17) and the end part (9) has a bore (18, 28; 17) with means (20; 29; 42; 62) for the releasable engagement for the purpose of transmitting a force in the axial direction, the bores (16; 17; 18, 28) being aligned axially relative to one another in the direction of the shaft when the parts (1; 5; 9) have been joined together, and in that a tie rod (15) having an engagement element is provided for the releasable engagement in the means (20; 29; 42; 62), and the tie rod (15) projects beyond the top part (1) when the parts of the prosthesis have been joined together.

2. Prosthesis kit according to Claim 1, characterised in that the means for the releasable engagement and the corresponding engagement element of the tie rod (15) form an internal thread (20; 29; 42; 62) or an external thread (19), the two parts of a bayonet coupling, or at least one element which can be spread apart from the tie rod (15) with a corresponding counter-surface.

3. Prosthesis kit according to one of the preceding claims, characterised in that, on the side opposite a cone (3 or 7) or a bore (16 or 17), the top part (1) and/or at least one intermediate part (5) has a contact surface (23) which surrounds the opening of the bore (16 or 17).

4. Prosthesis kit according to Claim 3, characterised in that a sleeve (21) is provided which can be pushed over the tie rod (15) and whose cross-section is adapted to the contact surface (23).

5. Prosthesis kit according to one of the preceding claims, characterised in that the tie rod (15) has an external thread (22) at its end opposite the engagement element.

6. Prosthesis kit according to one of the preceding claims, characterised in that the tie rod (15) has a region (24) with a reduced cross-section, the tensile strength of this region corresponding to a force which is sufficient to set the connecting cones of the top, end and intermediate parts (1, 5 and 9) in such a way that they are secured against loosening in the implanted state.

7. Prosthesis kit according to Claim 6, characterised in that an external thread (25) is provided near to the region with the reduced cross-section at the longer end of the tie rod (15).

8. Prosthesis kit according to Claim 7, characterised in that the engagement element, constructed as an external thread (19) on the tie rod (15), has an opposite direction of spiral to the external thread (25).

9. Prosthesis kit according to one of the preceding claims, characterised in that the end part (9) has an adjustment device (40; 30, 41; 40, 60, 61) with which movable elements (32, 33; 34, 35; 36, 37) of the end part (9) can be pressed apart when the shaft prosthesis has been inserted and can be clamped to the wall of the marrow cavity of the bone receiving the shaft.

10. Prosthesis kit according to Claim 9, characterised in that the adjustment device has a clamping element (30) arranged in the through-bore (16, 17, 28) to transmit a force in the axial direction.

11. Prosthesis kit according to Claim 9 or 10, characterised in that the movable elements of the end part (9) consist of shaft parts (32, 33; 36, 37) which can be spread apart with a cross-section which varies in the axial direction, and in that the clamping element (30) is connected to a bolt or wedge (40) which is movable in the axial direction and is arranged between the shaft parts (32, 33; 36, 37) which can be spread apart.

12. Prosthesis kit according to Claim 11, characterised in that the shaft parts (32, 33) which can be spread apart can be spread apart at the free end (12) of the shaft.

13. Prosthesis kit according to Claim 11, characterised in that the shaft parts (36, 37) which can be spread apart can be spread apart counter to the insertion direction of the shaft end (12).

14. Prosthesis kit according to Claim 9 or 10, characterised in that the movable elements of the end part (9) consist of resilient elements (34, 35) which are connected to one another at the upper and lower ends of the end part (9), and in that the clamping element (30) can be attached to the lower end of the resilient elements (34, 35) in such a way that the resilient elements (34, 35) can be bent apart when the clamping element (30) moves in the axial direction.

15. Prosthesis kit according to one of the preceding Claims 9 to 14, characterised in that the clamping element (30) consists of a thread pull or wire pull which is guided through a bore in the tie rod (15) and can be fixed in the region of the top part (1) of the shaft prosthesis.

16. Prosthesis kit according to one of the preceding Claims 9 to 14, characterised in that the clamping element (30) consists of a connecting rod or forcing lever which can be connected to a means provided in the bolt or wedge (40) for the releasable engagement (29).

17. Prosthesis kit according to Claim 14, characterised in that the clamping element (30) consists of a connecting rod which can be connected to a means which can be attached to the lower end of the resilient elements (34, 35) for the releasable engagement (42).

18. Prosthesis kit according to one of the preceding claims, characterised in that the clamping element (30) is the tie rod (15).

## Revendications

1. Jeu de pièces pour une prothèse à tige, comprenant une partie tête (1) munie d'une sphère d'articulation et une partie terminale (9) pouvant être ancrée dans le canal médullaire de l'os, l'une de ces parties possédant un tenon conique (8) et l'autre partie un perçage conique (3), et dans lequel il est prévu entre ces deux parties au moins une Partie intermédiaire (5) qui est adaptée au tenon (8) et/ou au perçage (3),
caractérisé
en ce que la partie tête (1) et la partie intermédiaire (5) présentent un perçage traversant (16 ; 17) et la partie terminale (9) présente un perçage (18, 28 ; 17), ainsi que des moyens (20 ; 29 ; 42 ; 62) servant à établir une prise démontable pour transmettre une force dans la direction axiale, les perçages (16 ; 17 ; 18, 28) étant alignés axialement les uns sur les autres, selon la direction de la tige, lorsque les parties (1 ; 5 ; 9) sont assemblées, et
en ce qu'un tirant (15) muni d'un élément de prise est prévu pour établir une prise démontable dans les moyens (20 ; 29 ; 42 ; 62), et le tirant (15) émerge de la partie tête (1) lorsque les parties de la prothèse sont assemblées.

2. Prothèse en plusieurs pièces selon la revendication 1, caractérisée en ce que les moyens servant à établir la prise démontable et l'élément de prise correspondant du tirant (15) formant respectivement un filetage intérieur (20 ; 29 ; 42 ; 62) et un filetage extérieur (19), ou les deux éléments d'un accouplement à baïonnette, ou encore au moins un élément expansible pouvant être dilaté sur le tirant (15), complété d'une surface conjuguée correspondante.

3. Prothèse en plusieurs pièces selon une des revendications précédentes, caractérisée en ce que la partie tête (1) et/ou au moins une partie intermédiaire (5) présentent, sur le côté qui est à l'opposé d'un cône (3 ou 7) ou d'un perçage (16 ou 17), une surface de portée (23) qui entoure l'ouverture du perçage (16 ou 17).

4. Prothèse en plusieurs pièces selon la revendication 3, caractérisée en ce qu'il est prévu un manchon (21) pouvant être emmanché sur le tirant (15) et dont la section est adaptée à la surface de portée (23).

5. Prothèse en plusieurs pièces selon une des revendications précédentes, caractérisée en ce que le tirant (15) présente un filetage extérieur (22) à son extrémité qui est à l'opposé de l'élément de prise.

6. Prothèse en plusieurs pièces selon une des revendications précédentes, caractérisée en ce que le tirant (15) possède une région (24) de section transversale réduite, la résistance à la traction de cette région correspondant à une force qui est suffisante pour mettre les cônes d'assemblage de la partie tête, de la partie terminale et de la partie intermédiaire (1, 5 et 9) de manière qu'elles soient bloquées et ne puissent pas se desserrer dans l'état implanté.

7. Prothèse en plusieurs pièces selon la revendication 6, caractérisée en ce qu'un filetage extérieur (25) est prévu sur l'extrémité longue du tirant (15) dans la région de section réduite.

8. Prothèse en plusieurs pièces selon la revendication 7, caractérisée en ce que l'élément de prise formé par un filetage extérieur (19) sur le tirant (15) présente un pas inverse de celui du filetage extérieur (25).

9. Prothèse en plusieurs pièces selon une des revendications précédentes, caractérisé en ce que la partie terminale (9) présente un dispositif de commande (40 ; 30, 41 ; 40, 60, 61) à l'aide duquel des éléments mobiles (32, 33 ; 34, 35 ; 36, 37) de la partie terminale (9) peuvent être écartés et bloqués par serrage contre la paroi du canal médullaire de l'os recevant la tige, après la mise en place de la prothèse à tige.

10. Prothèse en plusieurs pièces selon la revendication 9, caractérisé en ce que le dispositif de commande comprend un élément de serrage (30) disposé dans le perçage traversant (16, 27, 28) pour transmettre une force dans la direction axiale.

11. Prothèse en plusieurs pièces selon la revendication 9 ou 10, caractérisée en ce que les éléments mobiles de la partie terminale (9) sont composés de parties (32, 33 ; 36, 37) de la tige qui peuvent s'écarter l'une de l'autre, et qui possèdent une section transversale variable dans la direction axiale, et en ce que l'élément de serrage (30) est relié à une cheville ou à un coin (40) mobile dans la direction axiale, qui est disposé entre les parties (32, 33 ; 36, 37) de la tige qui peuvent s'écarter l'une de l'autre.

12. Prothèse en plusieurs pièces selon la revendication 11, caractérisée en ce que les parties (32, 33) de la tige qui peuvent s'écarter l'une de l'autre peuvent s'écarter l'une de l'autre à l'extrémité libre (12) de la tige.

13. Prothèse en plusieurs pièces selon la revendication 11, caractérisée en ce que les parties (36, 37) de la tige qui peuvent s'écarter l'une de l'autre peuvent s'écarter l'une de l'autre en sens inverse du sens d'introduction de l'extrémité (12) de la tige.

14. Prothèse en plusieurs pièces selon la revendication 9 ou 10, caractérisée en ce que les éléments mobiles de la partie terminale (9) sont constitués par des éléments élastiques (34, 35) qui sont reliés entre eux à l'extrémité supérieure et à l'extrémité inférieure de la partie terminale (9), et en ce que l'élément de serrage (30) peut être fixé à l'extrémité inférieure des éléments élastiques (34, 35) de manière que, lorsque cet élément de serrage (30) se déplace dans la direction axiale, les éléments élastiques (34, 35) puissent fléchir en s'écartant l'un de l'autre.

15. Prothèse en plusieurs pièces selon une des revendications 9 à 14 précédentes, caractérisée en ce que l'élément de serrage (30) est composé d'un filament ou d'un fil métallique enfilé dans un perçage du tirant (15) et qui peut être bloqué dans la région de la partie de tête (1) de la prothèse à tige.

16. Prothèse en plusieurs pièces selon une des revendications 9 à 14 précédentes, caractérisée en ce que l'élément de serrage (30) est composé d'une tringle de traction ou de compression, qui peut être reliée à un moyen prévu dans la cheville ou le coin (40) pour établir la prise démontable (29).

17. Prothèse en plusieurs pièces selon la revendication 14, caractérisée en ce que l'élément de serrage (30) est constitué par une tringle de traction qui peut être reliée, pour l'établissement de la prise démontable (42), à un moyen qui peut être fixé à l'extrémité inférieure des éléments élastiques (34, 35).

18. Prothèse en plusieurs pièces selon une des revendications précédentes, caractérisée en ce que l'élément de serrage (30) est constitué par le tirant (15).
